Europäisches Patentamt

European Patent Office

Office européen des brevets

(11) Publication number: **0 323 040 B1**

## EUROPEAN PATENT SPECIFICATION

(45) Date of publication of patent specification: **26.08.92**    (51) Int. Cl.⁵: **A61F  13/15**

(21) Application number: **88311394.6**

(22) Date of filing: **01.12.88**

(54) **Disposable diaper having shirred ears.**

(30) Priority: **04.12.87 US 128790**

(43) Date of publication of application:
**05.07.89 Bulletin  89/27**

(45) Publication of the grant of the patent:
**26.08.92 Bulletin  92/35**

(84) Designated Contracting States:
**DE ES FR GB IT NL SE**

(56) References cited:
**EP-A- 0 151 348       US-A- 2 905 581
US-A- 3 912 565       US-A- 4 381 781
US-A- 4 527 990       US-A- 4 655 760**

(73) Proprietor: **MINNESOTA MINING AND MANU-
FACTURING COMPANY
3M Center, P.O. Box 33427
St. Paul, Minnesota 55133-3427(US)**

(72) Inventor: **Wood , Leigh E. c/o Minnesota Min-
ing and
Manufacturing Company 2501 Hudson Road
St. Paul Minnesota 55144-1000(US)**
Inventor: **Zoia, Anthony J. c/o Minnesota Min-
ing and
Manufacturing Company 2501 Hudson Road
St. Paul Minnesota 55144-1000(US)**

(74) Representative: **Baillie, Iain Cameron et al
c/o Ladas & Parry, Altheimer Eck 2
W-8000 München 2(DE)**

# Description

## Background of the Invention

### Field of the Invention

The invention concerns contoured disposable diapers, especially diapers having stretchable and elastically contractable leg cuffs and waist bands. The invention particularly involves the problem of attaining improved fit to the wearer at insignificantly increased cost of manufacture compared to diapers now on the market.

### Description of the Related Art

Typically the body of a disposable diaper is substantially nonstretchable and has three major elements: a liquid permeable topsheet to be placed against the wearer's body, a liquid impermeable backsheet to form the face of the diaper, and an absorbent element interposed between the topsheet and backsheet. At the present time, those three elements are provided by three separate sheets, although it has been proposed to incorporate the absorbent and topsheet elements into a single sheet.

Most disposable diapers are contoured or form-fitting and their leg cuffs and waistbands are elastically contractable as illustrated in U.S. Pat. No. 4,515,595 (Kievet et al.). Although the elastic waistbands of the Kievet et al. patent extend completely across both ends of the diaper, it is more common for the waist elastic to extend only part way across each end as in U.K. Pat. Application GB 2,156,656 (Pomplun et al.). In a contoured disposable diaper, the ends of the waist portions are flared out to form ears, and typically an adhesive-bearing fastening tab is attached to the back ears.

The fastening tabs of most contoured diapers are aligned with the substantially nonstretchable body of the diaper, as in the above-discussed patent and patent application. Forces applied by a user to pull the diaper snugly around the buttocks often result in tearing at the ears. In French Pat. Publication No. 2,583,620 (Courtray) which was laid open December 25, 1986, the fastening tabs are aligned with an elastic waist band in order to make the diaper leakproof at the waist.

In the process of manufacturing disposable diapers, tension is typically maintained longitudinally, and elastic strips can be put into the leg cuffs in a stretched condition without difficulty, but the same is not true with waist strips which extend transversely where there is no tension. Accordingly, the waist of the diaper usually is made stretchable and elastically contractable by adhering to the backsheet a flat strip of heat-elasticizable material, i.e., a material which is heat-shrinkable and then can be stretched elastically to its original unshrunk dimensions. Then, after cutting out the diaper, the diaper is heated to shrink the heat-elasticizable material, thus shirring the waist band. When the heat-elasticizable material is a plastic film which also is heat-sealable, it can be adhered to the backsheet either adhesively or by being heat-sealed to the backsheet.

A preferred heat-elasticizable material for shirring the waist is disclosed in U.S. Pat. No. 4,552,795 (Hansen et al.). Before being shirred, it comprises a substantially flat inelastic web to which has been heat-bonded a plurality of parallel elastomeric strands that have been extended to at least about three times their relaxed length. The strand-bearing web can be coated with a layer of pressure-sensitive adhesive to adhere it to the backsheet.

Instead of the waist band being stretchable and elastically contractable, some disposable diapers employ stretchable fastening tapes as in U.S. Pat. No. 3,800,796 (Jacob). Although the diaper of the Jacob patent is not contoured, stretchable fastening tapes have been used on disposable diapers which are contoured.

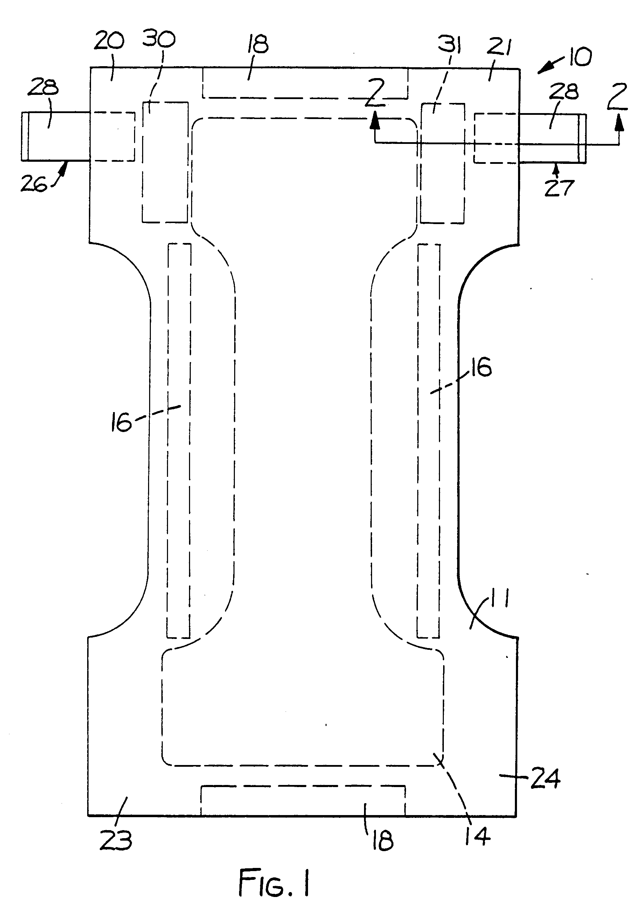

U.S. Pat. No. 4,381,781 (Sciaraffa et al.) says that when there is an elastic waist strip, "the folding of the diaper waist end, which must take place after the continuous web is cut into individual diapers, is extremely difficult to accomplish. This is because the leg elastic will retract the individual diaper lengthwise if it is not held entirely flat while moving rapidly along the production line until packaging and the folding operation while simultaneously holding the diaper flat cannot be done readily" (col. 1, lines 35-47). The Sciaraffa et al. patent avoids an elastic waist strip, by cutting, as shown in Fig. 1, "openings 70 and 72 through the joined topsheet 2 and backsheet 4. The openings 70 and 72 intersect the waist edge 18 such that there is no diaper material between each of the openings 70 and 72 and the waist edge 18.... Elastic layer material such as layers 78 and 80 is respectively disposed in the openings 70 and 72 and affixed to either the topsheet 2 or backsheet 4, or both, so that the layers 78 and 80 become an integral part of the diaper as shown in FIG. 7" (col. 4. lines 7-19). The "elastic layers 78 and 80 may respectively be located entirely or partially within the ears 42 and 44 or entirely laterally inward of the ears 42 and 44" (col. 4, lines 41-44). "Due to the inelasticity of the topsheet 2 and backsheet 4, or both, most of the transverse tensile stress is applied to the elastic layer 80 causing it to stretch and provide a snug fit between the skin of the wearer and the waist areas 8 and 10" (col. 5. lines 5-10).

The Sciaraffa et al. patent says: "The attaching of the elastic material to the back sheet and/or top sheet of the diaper requires only cutting the sheets as they move in web form, cutting the elastic material, and pressing the elastic material against one of the sheets. These manufacturing steps are all readily accomplished in conjunction with the usual high speed diaper production lines" (col. 2, lines 32-38). No mention is made of the need to maintain good registration and probable difficulties in attempting to do so. It is believed that the diaper of the Sciaraffa et al. patent has not appeared on the market.

Summary of the Invention

The invention provides a contoured disposable diaper which can be pulled snugly around the buttocks and the waist merely by applying ordinary fingertip force to the fastening tabs. The resulting improved fit is achieved while minimizing the problem of accidental tearing and is accompanied by both better appearance and enhanced performance. The novel diaper is believed to be the first disposable diaper which provides those advantages and yet can be manufactured at substantially the same cost as disposable diapers now on the market. Like those now on the market, the body of the diaper of the invention is generally substantially nonstretchable and has a liquid permeable topsheet, a liquid impermeable backsheet, and an absorbent element interposed between the topsheet and backsheet. As noted above, the absorbent and topsheet elements might be incorporated into a single sheet. At least one of the topsheet and backsheet is flared to form a pair of back ears. A fastening tab, which preferably is substantially nonstretchable, is attached to each of the back ears.

The diaper of the invention differs from prior contoured disposable diapers in that at least one of its back ears is elastically shirred in an area aligned with its fastening tab to permit it to be stretched elastically at least one cm under ordinary fingertip force applied to the fastening tab. The shirred area, in an infant diaper of average size, should extend over a height of at least 3 cm (preferably from 4 to 8 cm). For the smallest infant diapers, the shirring height should be at least 2 cm, and for an adult diaper of average size, the shirring height should be at least 6 cm. By "height" is meant the direction between the waist and the crotch of the diaper. "Ordinary fingertip force" may range from about 200 to 2000 grams of force.

The elastic shirring preferably is provided by adhering to the backsheet or topsheet, or both, a heat-elasticizable material, preferably including a plurality of parallel elastomeric strands as in the above-cited Hansen et al. patent. The force necessary to stretch the elastically shirred ear at least one cm can be controlled by the modulus of the heat-elasticizable material in its heat-elasticized state, its width, its thickness, or combinations thereof. Upon stretching an elastically shirred ear close to its elastic limit, the user feels an abrupt increase in force necessary to continue to elongate the ear. The diaper should be designed so that when this abrupt increase is felt, sufficient tension has been applied to realize a snug fit around the wearer's buttocks and waist, and the user should realize that additional force is unnecessary. This should greatly reduce the hazard of tearing of the ear as compared to disposable diapers now on the market. In this invention, the abrupt increase in tension is felt at from 500 to 1500 grams of force for an infant diaper of average size.

Because the novel diaper can be easily pulled snugly around the buttocks and waist, there should be no need for an elastically contractable waist band, thus saving the cost of elasticizing the waist.

The diaper of the invention can be manufactured with equipment now in widespread use, e.g., as shown and described in U.S. Pat. No. 4,563,185 (Reiter). This can be accomplished merely by adding to the line one station at which small pieces of heat-elasticizable material are adhered to the backsheet adjacent at least one of its edges. When using equipment including a station at which a strip of heat-elasticizable material is applied at the waist, that station can be modified so that instead of applying material at the waist, a small piece of heat-elasticizable material is applied at each back ear. Alternatively, that station can be modified to apply a U-shaped strip across the waist, with the ends of the U providing the elastic shirring across the ears. Because the cutting of the U-shaped strips might involve greater waste of raw material than the application of three separate pieces, it may be more economical to modify the station to apply three pieces simultaneously. The back ears become elastically shirred at the end of the assembly portion of the diaper line where the diapers are exposed to heat, there used to shir the waist band as described in the above-cited Reiter patent.

Preferably the shir across each back ear is at least 4 cm in height in order to spread the tension applied to the fastening tabs across a large area at each side of the rear of the diaper. This ensures a snug fit around the buttocks and waist merely by applying ordinary fingertip pressure to the fastening tabs. Before being heated, the heat-elasticizable material should be at least 2 cm, preferably at least 3 cm, in width in the circumferential direction of the diaper in order to permit the ear to be stretched at least one cm under ordinary fingertip force. It may be economically wasteful in infant diapers for the heat-elasticizable material to be more than 6 cm in width before the shirring.

As compared to the need to maintain good registration in making the diaper of the above-discussed Sciaraffa et al. patent, the pieces of heat-elasticizable material applied to the back ears of the novel diaper need not be applied with precision, just as precision is unnecessary in applying heat-elasticizable waist strips in current diaper manufacture. In order to allow substantial errors in applying pieces of the heat-elasticizable material to the ears, their application preferably is programmed to leave a space of at least 0.5 cm between the piece and the edge of the waist, thus ensuring that no piece will extend into the front waist region of the adjacent diaper. Except for economy, it is unnecessary to leave a space between the heat-elasticizable material and the leg openings if the heat-elasticizable material is applied before the leg openings are cut out.

In order to keep costs to a minimum by conserving material, the heat-elasticizable material preferably extends only about half the height of each back ear, e.g., from 4 to 8 cm, as compared to the height of the ears of an infant diaper of average size being about 10-15 cm. Preferably the heat-elasticizable material is at the center of the height of the ear, and the fastening tab is aligned with the piece of heat-elasticizable material. When the ear is elastically shirred by exposing the heat-elasticizable material to heat, the ear becomes inelastically shirred across the rest of its height.

The Drawing

The invention may be more understandable by reference to the drawing, all figures of which are schematic, wherein:

Fig. 1 is a plan view of a first contoured disposable diaper of the invention in a flat condition, partly broken away;

Fig. 2 is a cross section along line 2-2 of Fig. 1;

Fig. 3 is a cross section similar to that of Fig. 2 except after shirring;

Fig. 4 is a plan view of the ear and waist portions of a second contoured disposable diaper of the invention in a flat condition, partly broken away;

Fig. 5 is a perspective view of the diaper of Figs. 1-3 in the configuration it would assume when being worn by a person; and

Fig. 6 shows a cycled stress-strain curve for the shirred ear of a preferred diaper of the invention.

Detailed Description

The body of the diaper 10 shown in Figs. 1-3 and 5 is made up of a liquid permeable topsheet 11 to be placed against the wearer's body, a liquid impermeable backsheet 12 to form the face of the diaper, and an absorbent element 14 interposed between the topsheet and backsheet. Also interposed between the topsheet and backsheet are elastically contractable strips 16 and 18 at the leg cuffs and waist bands, respectively. As in most contoured disposable diapers now on the market, the elastically contractable strips 16 at the leg cuffs are applied in a stretched condition while those 18 at the waist bands are heat-elasticizable material that does not become elastically contractable until it has been heat-shrunk. The ends of the waist portions of the diaper 10 are flared out to form back ears 20 and 21 and front ears 23 and 24. Attached to the back ears 20 and 21 are fastening tabs 26 and 27, respectively. Each fastening tab bears a layer of pressure-sensitive adhesive 28 by which it can be secured to the front of the diaper. Pieces 30 and 31 of heat-elasticizable material are adhered to the backsheet 12 at each of the back ears 20 and 21, respectively, by a layer of pressure-sensitive adhesive 32.

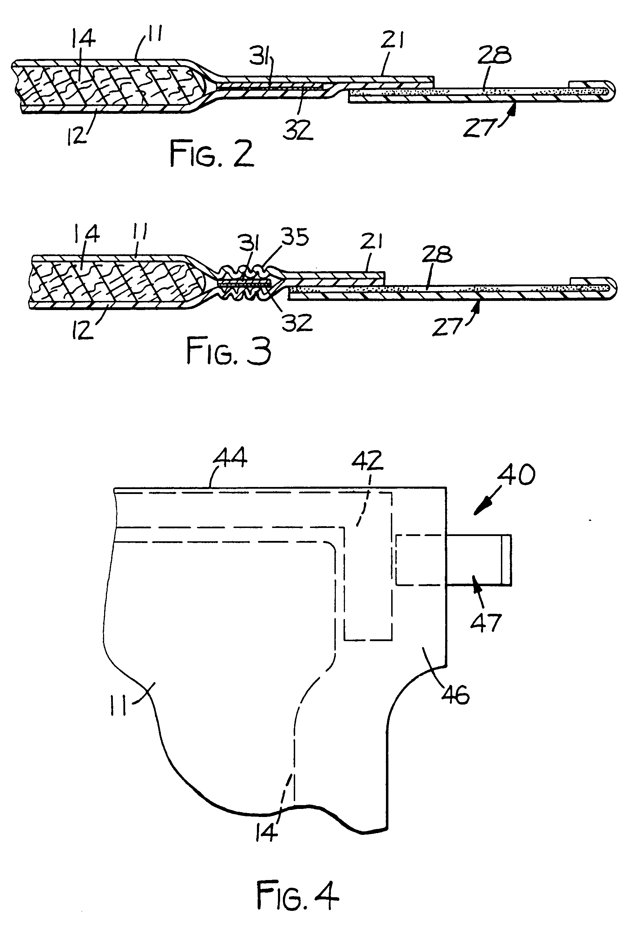

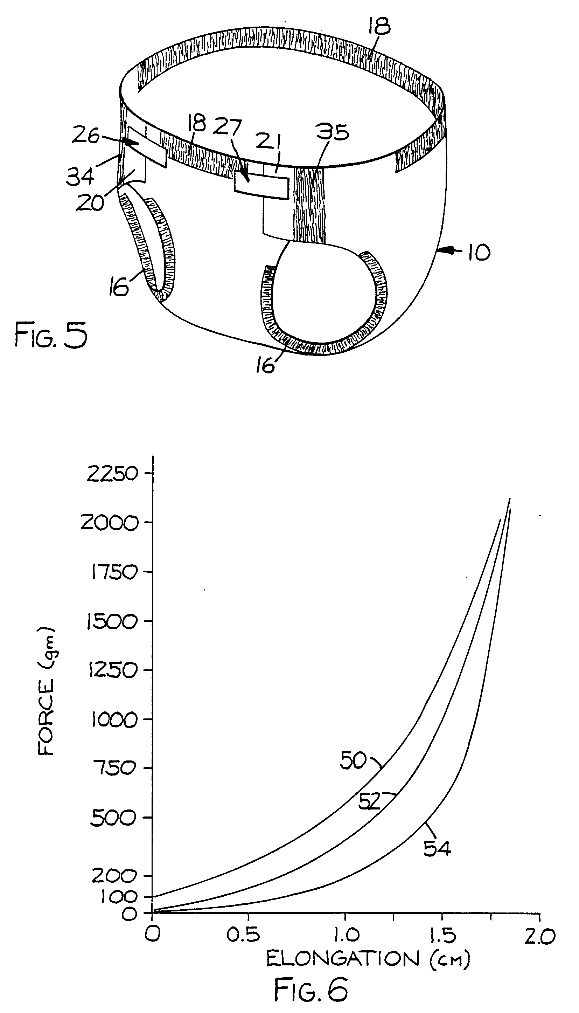

After the diaper 10 has been formed flat as shown in Figs. 1 and 2, it is heated to heat-shrink the waist strips 18 and the pieces 30 and 31 of heat-elasticizable material. Shrinkage of the latter causes elastic shirring 34 and 35 of the ears 20 and 21, respectively, as best seen in Figs. 3 and 5.

The diaper 40 shown fragmentally in Fig. 4 is identical to that of Fig. 1-3 and 5 except that it employs a single U-shaped piece 42 of heat-elasticizable material instead of two separate pieces. When the diaper 40 is heated, shrinkage of the U-shaped piece 42 will shir both the waist band 44 and the back ear 46. The diaper 40 has fastening taps 47 similar to those of the diaper 10.

Suitable heat-elasticizable materials for use in providing the heat-activated elastic shirring of the back ears 20 and 21 includes elastic films such as flexible polyurethanes as described in U.S. Pat. No. 3,912,565 (Koch et al.), plasticized vinyl chloride polymers as described in U.S. Pat. No. 3,819,401 (Massengale et. al.), copolymers of alternating polyamide and polyether blocks as described in U.K. Pat. Appl. No. 2,160,437A (Matray et al.), and other block copolymers as described in U.S. Pat. No. 3,639,917 (Althouse). Also suitable are the elastic composite films of U.S. Pat. No. 4,652,487 (Mormon), U.S. Pat. No. 4,657,802 (Mormon), and U.S. Pat. No. 4,640,859 (Hansen et al.).

The piece of heat-elasticizable material should shrink at least 30% when heated, thus permitting the back ear to be stretched elastically at least 30% the width of the piece of heat-elasticized material. Preferably the heat-elasticized material shrinks about 50%.

Example

The heat-elasticizable material used in this Example is a flat inelastic web to which had been heat-bonded a plurality of parallel elastomeric strands extended to at least about three times their relaxed length as disclosed in the above-cited Hansen et al. patent. This material was cut to pieces having a length of 2 inches (5 cm) and a width of 2 inches (5 cm). To each piece was laminated a layer of pressure-sensitive adhesive.

The topsheet of a commercially available diaper ("Luvs" brand disposable diaper from Procter and Gamble Co.) was mechanically separated from the backsheet in a rear end region. The adhesive side of a piece of heat-elasticizable material was then adhered to the inside face of the backsheet of the diaper, after which the rear end was reassembled, reattaching the topsheet to the backsheet by using a thin strip of pressure-sensitive adhesive. The width and the strands of the piece of heat-elasticizable material were oriented in the circumferential direction of the diaper (transverse to the height of the ear). The diaper was then placed in a current of circulatory hot air (200°F, 93°C) for 40 seconds. The ear portion of the diaper shirred, and the width of the piece of heat-elasticizable material was reduced to 1.06 inches (2.7 cm), a shrinkage of 47%.

A cycled stress-strain curve for the shirred ear of the diaper of the Example is shown in Fig. 6. This curve was made in an Instron tensile tester, the lower jaw of which extended across the full width of the fastening tape while the upper jaw (7.5 cm in width) clamped the center of the diaper from the top toward the crotch. In doing so, the shirred ear was slightly stretched to ensure against any slack. The upper jaw was moved away from the lower jaw at a speed of 10 inches (25 cm) per minute. In Fig. 6, line 50 shows the first pull, line 52 shows the second and subsequent pulls, and line 54 shows the relaxation curve. Lines 50 and 52 indicate that when stretching of the shirred ear approaches its elastic limit, a user of the diaper would be warned that substantially increased force might accidentally tear the ear.

While the diaper shown in the drawing employs pressure-sensitive adhesive fastening tabs, other fastening means such as snaps or hook-and-loop fasteners are also useful in diapers of the invention.

Although the Example and the drawings illustrate the use of adhesive to affix heat-elasticizable material to either the topsheet or backsheet of the diaper of the present invention, alternate methods such as heat bonding and sonic welding can also be employed.

**Claims**

1. A contoured diaper comprising a liquid permeable topsheet(11), a liquid impermeable backsheet (12), and an absorbent element (14) interposed between the topsheet (11) and backsheet (12), at least one of the topsheet and backsheet being flared to form a pair of back ears (20, 21), each back ear having a fastening tab (26, 27), characterised by at least one of the back ears (20, 21) being elastically shirred (34, 35) in an area aligned with its fastening tab (26, 27), which shirring extends over a height of at least 3 cm and permits the back ear (20, 21) to be stretched elastically at least one cm under ordinary fingertip force applied to the fastening tab, said tab (26, 27) exhibiting an abrupt increase in tension at from 500 to 1500 grams of force.

2. Diaper as defined in claim 1 wherein both back ears (20, 21) are shirred.

3. Diaper as defined in claim 2 wherein the shir (34, 35) across each back ear (20, 21) is at least 4 cm in height.

4. Diaper as defined in claim 3 wherein the shir (34, 35) is provided by a piece of heat-elasticisable material (30, 31) adhered to at least one of the topsheet (11) and backsheet (12).

5. Diaper as defined in claim 4 wherein said back ear (20, 21) can be stretched elastically at least 30% of the width of the piece of heat-elasticisable material (30, 31).

6. Diaper as defined in claim 4 wherein the heat-elasticisable material (30, 31) at each ear is contiguous with a strip of the heat-elasticisable material extending across the waistband to form, for example, a U-shaped piece (42).

7. Diaper as defined in claim 4 wherein the heat-elasticisable material (30, 31) at each ear are separate, spaced, elements adjacent opposite edges of said topsheet (11) and backsheet (12).

8. Diaper as defined in claim 4 wherein the heat-elasticisable material (30, 31) bears a layer of pressure-sensitive adhesive (32) by which it is adhered to the backsheet (12).

9. Diaper as defined in claim 8 wherein the heat-elasticisable material (30, 31) before being shirred comprises a substantially flat inelastic web to which is heat-bonded a plurality of parallel elastomeric strands extended to at least three times their relaxed length.

10. Method of making the contoured diaper of claim 1 wherein great lengths of said topsheet (11), backsheet (12) and absorbent element are moved in the direction of their lengths, said method comprising the steps of:

1) adhering to at least one of said topsheet (11) and backsheet (12), adjacent at least one of its edges, a piece of heat-elasticisable material (30),

2) continuously bonding said elements together into a coherent body,

3) cutting out individual diapers from said body to form a back ear (20, 21) at each adhered piece of heat-elasticisable material, and

4) heating the diapers to shrink said piece of heat-elasticisable material and thus elastically shir (34, 35) the body of each diaper at a back ear (20, 21), characterised by a pair of spaced apart flat pieces of heat-elasticisable material (30, 31) which exhibit an abrupt increase in tension at 500-1500 grams of force when said back ear (20, 21) is stretched.

11. Method as defined in claim 10, in step 1) of which each piece of heat-elasticisable material (30, 31) are adhered in pairs adjacent opposite edges of said topsheet (11) and backsheet (12).

12. Method as defined in claim 10, in step 1) of which each piece of heat-elasticisable material (30, 31) is U-shaped (42) and is adhered with the ends of each U adjacent opposite edges of said backsheet (12).

13. Method as defined in claim 10 wherein the heat-elasticisable material shrinks at least 30% in step 4).

14. Method as defined in claim 10 wherein said flat pieces of heat-elasticisable material (30, 31) comprise a substantially flat inelastic web to which is heat bonded a plurality of parallel elastomeric strands extended to at least about three times their relaxed length.

15. Method as defined in claim 10 wherein said flat pieces of heat-elasticisable material (30, 31) bear a layer of pressure-sensitive adhesive (32) by which it is adhered to at least one of said topsheet and backsheet.

**Patentansprüche**

1. Körperangepaßte Windel mit einer flüssigkeits-durchlässigen Oberlage (11), einer flüssigkeits-undurchlässigen Unterlage (12) und einem aufsaugenden Element (14), das zwischen der Oberlage (11) und der Unterlage (12) angeordnet ist, wobei mindestens eine der Oberlage und Unterlage zur Bildung eines Bundohrenpaares (20, 21) ausgestellt ist und jedes Bundohr ein Verschlußband (26, 27) aufweist, **dadurch gekennzeichnet, daß** mindestens eines der Bundohren (20, 21) in einer Fläche elastisch gekräuselt (34, 35) ist, die mit seinem Verschlußband (36, 37) auf einer Linie liegt, die Kräuselung sich mindestens über eine Höhe von 3 cm erstreckt und dem Bundohr (20, 21) eine elastische Streckung von mindestens einem Zentimeter unter normalem, auf den Verschlußstreifen ausgeübten Fingersptizendruck ermöglicht, wobei das Band (26, 27) einen sprunghaften Spannungzuwachs von 500 bis 1500 Pond zeigt.

2. Windel nach Anspruch 1, in welcher beide Bundohren (20, 21) gekräuselt sind.

3. Windel nach Anspruch 2, in welcher die Kräuselung (34, 35) über jedes Bundohr (20, 21) mindesten 4 cm hoch ist.

4. Windel nach Anspruch 3, in welcher die Kräuselung (34, 35) mit einem Stück eines heißelastisierbaren Materials (30, 31) versehen ist, das mindestens auf einer der Oberlage (11) und Unterlage (12) aufgeklebt ist.

5. Windel nach Anspruch 4, in welcher das Bundohr (20, 21) um mindestens 30% der Breite des heißelastisierbaren Stück Materials (30, 31) gestreckt werden kann.

6. Windel nach Anspruch 4, in welcher das heißelastisierbare Material (30, 31) an jedem Ohr an einem Band des heißelastisierbaren Materials anliegt, das sich quer über den Bund erstreckt, um zum Beispiel ein U-förmiges Stück zu bilden.

7. Windel nach Anspruch 4, in welcher das heißelastisierbare Material (30, 31) an jedem Ohr separate, im Abstand angeordnete Elemente neben den gegenüberliegenden Rändern der Oberlage (11) und der Unterlage (12) aufweist.

8. Windel nach Anspruch 4, in welcher das heißelastisierbare Material (30, 31) eine selbstklebende Schicht (32) trägt, durch die es auf der Unterlage (12) aufgeklebt wird.

**9.** Windel nach Anspruch 8, in welcher das heißelastisierbare Material (30, 31), bevor es gekräuselt wird, ein im wesentlichen nichtelastisches Gewebe enthält, in das unter Hitze viele parallele, elastomere Fäden eingebunden werden, die mindestens auf das Dreifache ihrer entspannten Länge ausgedehnt sind.

**10.** Verfahren zur Herstellung der körperangepaßten Windel nach Anspruch 1, in welchem große Längen der Oberlage (11), Unterlage (12) und des aufsaugenden Elements in ihrer Längsrichtung bewegt werden, wobei das Verfahren die Schritte umfaßt:
1) Aufkleben eines Stücks heißelastisierbaren Materials (30) auf mindestens einer der Oberlage (11) und der Unterlage (12) neben mindestens einer ihrer Ränder,
2) Kontinuierliches Verbinden der Elemente miteinander zu einem zusammenhängenden Körper,
3) Ausschneiden einzelner Windeln aus dem Körper zur Bildung eines Bundohres (20, 21) an jedem aufgeklebten Stück heißelastisierbaren Materials (30, 31) und
4) Erhitzen der Windeln, um das Stück heißelastisierbaren Materials zu schrumpfen und damit jedes Windelstück an einem Bundohr (20, 21) elastisch zu kräuseln (34, 35), gekennzeichnet durch ein Paar im Abstand zueinander angeordneter Stücke heißelastisierbaren Materials (30, 31), die einen sprunghaften Spannungzuwachs von 500 bis 1500 Pond zeigen, wenn das Bundohr (20, 21) gestreckt wird.

**11.** Verfahren nach Anspruch 10, in dessen Schritt (1) jedes Stück des heißelastisierbaren Materials (30, 31) paarweise neben den gegenüberliegenden Rändern der Oberlage (11) und Unterlage (12) zusammengeklebt wird.

**12.** Verfahren nach Anspruch 10, in dessen Schritt (1) jedes Stück des heißelastisierbaren Materials (30, 31) eine U-Form (42) aufweist und mit den Enden jedes U neben den gegenüberliegenden Rändern der Unterlage (12) aufgeklebt wird.

**13.** Verfahren nach Anspruch 10, in welchem das heißelastisierbare Material in Schritt (4) mindestens 30% schrumpft.

**14.** Verfahren nach Anspruch 10, in welchem die flachen Stücke des heißelastisierbaren Materials (30, 31) ein im wesentlichen flaches, nichtelastisches Gewebe aufweisen, in das unter Hitze viele parallele, elastomere Fäden eingebunden werden, die mindestens auf das Dreifache ihrer entspannten Länge ausgedehnt sind.

**15.** Verfahren nach Anspruch 10, in welchem die flachen Stücke des heißelastisierbaren Materials (30, 31) eine selbstklebende Schicht (32) tragen, durch die sie an mindesten einer der Oberlage und Unterlage aufgeklebt werden.

## Revendications

**1.** Couche adaptée au corps humain comprenant une feuille de dessus (11) perméable à un liquide, une feuille arrière (12) imperméable au liquide, et un élément absorbant (14) disposé entre la feuille de dessus (11) et la feuille arrière (12), au moins une des feuilles de dessus et arrière étant évasée pour former une paire d'oreilles arrières (20, 21), chaque oreille arrière comportant une languette d'attache (26, 27), caractérisée en ce qu'au moins une des oreilles arrières (20, 21) est élastiquement contractée (34, 35) dans une zone alignée avec sa languette d'attache (26, 27), laquelle contraction s'étend sur une hauteur d'au moins 3 cm et permet à l'oreille arrière (20, 21) d'être étirée élastiquement d'au moins un centimètre sous une force d'extrémité de doigt ordinaire appliquée sur la languette d'attache, ladite languette d'attache (26, 27) présentant une augmentation brutale en tension de 500 à 1 500 grammes - force,

**2.** Couche selon la revendication 1, dans laquelle les deux oreilles arrières (20, 21) sont contractées.

**3.** Couche selon la revendication 2, dans laquelle la contraction (34, 35) en travers de chaque oreille arrière (20, 21) est prévue sur une hauteur d'au moins 4 cm.

**4.** Couche selon la revendication 3, dans laquelle la contraction (34, 35) est obtenue au moyen d'un morceau de matière (30, 31) rendue élastique thermiquement et qui adhère à au moins une des feuilles de dessus (11) et arrière (12).

**5.** Couche selon la revendication 4, dans laquelle ladite oreille arrière (20, 21) Peut être étirée élastiquement d'au moins 30 % de la largeur du morceau de matière rendue élastique thermiquement (30, 31).

**6.** Couche selon la revendication 4, dans laquelle la matière rendue élastique thermiquement (30, 31) de chaque oreille, est contigue à une bande de matière rendue élastique thermiquement s'étendant en travers de la ceinture pour former, par exemple, une pièce (42) en forme de U.

7. Couche selon la revendication 4, dans laquelle la matière rendue élastique thermiquement (30, 31) de chaque oreille est constituée par des éléments distincts et séparés, adjacents aux bords opposés de la feuille de dessus (11) et arrière (12).

8. Couche selon la revendication 4, dans laquelle la matière rendue élastique thermiquement (30, 31) porte une couche d'adhésif sensible à la pression (32) au moyen de laquelle elle adhère à la feuille arrière (12).

9. Couche selon la revendication 6, dans laquelle la matière rendue élastique thermiquement (30, 31), avant qu'elle ne soit contractée, comprend un support non élastique sensiblement plat auquel est liée par chaleur une pluralité de brins élastomériques et parallèles s'étendant sur au moins trois fois leur longueur à l'état relaxé.

10. Procédé de réalisation d'une couche adaptée selon la revendication 1, dans lequel de grandes longueurs desdites feuille de dessus (11), feuille arrière (12), et élément absorbant sont entraînées dans la direction de leur longueur, ledit procédé comprenant les étapes :

    1. de fixer un morceau de matière thermoélastique (30) sur an moins une desdites feuilles de dessus (11) et arrière (12), de façon adjacente à au moins un de ses bords,

    2. de lier de façon continue lesdits éléments entre eux pour constituer un ensemble cohérent,

    3. de découper des couches individuelles dans ledit ensemble pour former une oreille arrière (20, 21) dans chaque morceau de matière rendue élastique thermiquement qui a été fixé ; et

    4. de chauffer les couches pour rétracter ledit morceau de matière rendue élastique thermiquement et ensuite contracter élastiquement (34, 35) l'ensemble de chaque couche au niveau d'une oreille arrière (20, 21), caractérisé en ce qu'on étire une paire d'éléments plats et espacés de la matière rendue élastique thermiquement (30, 31) qui présentent une augmentation brutale en tension de 500 à 1 500 grammes - force lorsque ladite oreille (20, 21) est étirée.

11. Procédé selon la revendication 10, dans l'étape 1 duquel les morceaux de matière rendue élastique thermiquement (30, 31) sont fixés par paires de façon adjacente aux bords opposés desdites feuille de dessus (11) et feuille arrière (12).

12. Procédé selon la revendication 1, dans l'étape 1 duquel chaque morceau de matière rendue élastique thermiquement (30, 31) est en forme de U et il est fixé de façon que les extrémités de chaque U soient adjacentes aux bords opposés de ladite feuille arrière (12).

13. Procédé selon la revendication 10, dans lequel la matière rendue élastique thermiquement se rétracte d'au moins 30 % de l'étape 4.

14. Procédé selon la revendication 10, dans lequel les éléments plats de matière rendue élastique thermiquement comportent un support non élastique et sensiblement plat auquel est liée thermiquement une pluralité de brins élastomériques parallèles s'étendant sur au moins environ trois fois leur longueur relaxée.

15. Procédé selon la revendication 10, dans lequel les éléments plats de matière rendue élastique thermiquement (30, 31) portent une couche d'adhésif (32) sensible à la pression au moyen de laquelle ils adhèrent à au moins une desdites feuilles de dessus et arrière.

FIG. 1

FIG. 2

FIG. 3

FIG. 4

Fig. 5

FORCE (gm)

ELONGATION (cm)

Fig. 6